(19) **Europäisches Patentamt**
**European Patent Office**
**Office européen des brevets**

(11) **EP 1 107 743 B1**

(12) **EUROPEAN PATENT SPECIFICATION**

(45) Date of publication and mention
of the grant of the patent:
**27.06.2007 Bulletin 2007/26**

(51) Int Cl.:
**A61K 9/72** (2006.01)  **A61K 9/14** (2006.01)

(21) Application number: **99945175.0**

(22) Date of filing: **25.08.1999**

(86) International application number:
**PCT/US1999/019306**

(87) International publication number:
**WO 2000/010541 (02.03.2000 Gazette 2000/09)**

(54) **STABLE SPRAY-DRIED PROTEIN FORMULATIONS**

SPRÜHGETROCKNETE PROTEINFORMULIERUNGEN

FORMULATIONS PROTEIQUES ATOMISEES STABLES

(84) Designated Contracting States:
**AT BE CH CY DE DK ES FI FR GB GR IE IT LI LU MC NL PT SE**

(30) Priority: **25.08.1998 US 97796 P**

(43) Date of publication of application:
**20.06.2001 Bulletin 2001/25**

(60) Divisional application:
**06025536.1 / 1 767 195**

(73) Proprietor: **Advanced Inhalation Research, Inc.
Cambridge, MA 02139 (US)**

(72) Inventors:
• **EDWARDS, David, A.
Boston, MA 02115 (US)**
• **HRKACH, Jeffrey, S.
Cambridge, MA 02140 (US)**

(74) Representative: **Kirkham, Nicholas Andrew
Graham Watt & Co LLP
St Botolph's House
7-9 St Botolph's Road
Sevenoaks
Kent TN13 3AJ (GB)**

(56) References cited:
**EP-A- 0 317 120**    **EP-A- 0 655 237**
**WO-A-91/16882**    **WO-A-97/26863**
**WO-A-97/44012**    **WO-A-98/31346**

• **Y.-F. MAA ET AL.: "spray-drying of air-liquid interface sensitive recombinant human growth hormone" JOURNAL OF PHARMACEUTICAL SCIENCES, vol. 87, no. 2, February 1998 (1998-02), pages 152-159, XP000729419 Washington (US)**

Remarks:
The file contains technical information submitted after the application was filed and not included in this specification

**Description**

BACKGROUND OF THE INVENTION

Aerosols for the delivery of therapeutic agents to the respiratory tract have been described, for example, Adjei, A. and Garren, J. Pharm. Res., 7: 565-569 (1990); and Zanen, P. and Lamm, J.-W.J. Int. J. Pharm., 114: 111-115 (1995). The respiratory tract encompasses the upper airways, including the oropharynx and larynx, followed by the lower airways, which include the trachea followed by bifurcations into the bronchi and bronchioli. The upper and lower airways are called the conducting airways. The terminal bronchioli then divide into respiratory bronchioli which then lead to the ultimate respiratory zone, the alveoli, or deep lung. Gonda, I. "Aerosols for delivery of therapeutic and diagnostic agents to the respiratory tract," in Critical Reviews in Therapeutic Drug Carrier Systems, 6: 273-313 (1990). The deep lung, or alveoli, are the primary target of inhaled therapeutic aerosols for systemic drug delivery.

Inhaled aerosols have been used for the treatment of local lung disorders including asthma and cystic fibrosis (Anderson, Am. Rev. Respir. Dis., 140: 1317-1324 (1989)) and have potential for the systemic delivery of peptides and proteins as well. (Patton and Platz, Advanced Drug Delivery Reviews, 8: 179-196 (1992)). However, pulmonary drug delivery strategies present many difficulties for the delivery of macromolecules; these include protein denaturation during aerosolization, excessive loss of inhaled drug in the oropharyngeal cavity (often exceeding 80%), poor control over the site of deposition, lack of reproducibility of therapeutic results owing to variations in breathing patterns, the frequent too-rapid absorption of drug potentially resulting in local toxic effects, and phagocytosis by lung macrophages.

Considerable attention has been devoted to the design of therapeutic aerosol inhalers to improve the efficiency of inhalation therapies. Timsina et. al., Int. J. Pharm., 101: 1-13 (1995); and Tansey, I.P., Spray Technol. Market, 4: 26-29 (1994). Attention has also been given to the design of dry powder aerosol surface texture, regarding particularly the need to avoid particle aggregation, a phenomenon which considerably diminishes the efficiency of inhalation therapies. French, D.L., Edwards, D.A. and Niven, R.W., J. Aerosol Sci., 27: 769-783 (1996). Dry powder formulations ("DPFs") with large particle size have improved flowability characteristics, such as less aggregation (Visser, J., Powder Technology 58: 1-10 (1989)), easier aerosolization, and potentially less phagocytosis. Rudt, S. and R.H. Muller, J. Controlled Release, 22: 263-272 (1992); Tabata, Y. and Y. Ikada, J. Biomed Mater. Res., 22: 837-858 (1988). Dry powder aerosols for inhalation therapy are generally produced with mean geometric diameters primarily in the range of less than 5 $\mu$m, typically ranging from 1 to 5 $\mu$m. Ganderton, D., J Biopharmaceutical Sciences, 3: 101-105 (1992); and Gonda, I. "Physico-Chemical Principles in Aerosol Delivery," in Topics in Pharmaceutical Sciences 1991, Crommelin, D.J. and K.K. Midha, Eds., Medpharm Scientific Publishers, Stuttgart, pp. 95-115, 1992. Large "carrier" particles (containing no drug) have been co-delivered with therapeutic aerosols to aid in achieving efficient aerosolization among other possible benefits. French, D.L., Edwards, D.A. and Niven, R.W., J. Aerosol Sci., 27: 769-783 (1996).

The human lungs can remove or rapidly degrade, for example by hydrolysis or hydrolytic cleavage, deposited aerosols over periods ranging from minutes to hours. In the upper airways, ciliated epithelia contribute to the "mucociliary escalator" by which particles are swept from the airways toward the mouth. Pavia, D. "Lung Mucociliary Clearance," in Aerosols and the Lung: Clinical and Experimental Aspects, Clarke, S. W. and Pavia, D., Eds., Butterworths, London, 1984. Anderson, Am. Rev. Respir. Dis., 140: 1317-1324 (1989). In the deep lungs, alveolar macrophages are capable of phagocytosing particles soon after their deposition. Warheit, M.B. and Hartsky, M.A., Microscopy Res. Tech., 26: 412-422 (1993); Brain, J.D., "Physiology and Pathophysiology of Pulmonary Macrophages," in The Reticuloendothelial System, S.M. Reichard and J. Filkins, Eds., Plenum, New York, pp. 315-327, 1985; Dorries, A.M. and Valberg, P.A., Am. Rev. Resp. Disease 146: 831-837 (1991); and Gehr, P., Microscopy Res. and Tech., 26: 423-436 (1993). As the diameter of particles exceeds 3 $\mu$m, there is increasingly less phagocytosis by macrophages. Kawaguchi, H., Biomaterials 7: 61-66 (1986); Krenis, L.J. and Strauss, B., Proc. Soc. Exp. Med, 107: 748-750 (1961); and Rudt, S. and Muller, R.H., J Contr. Rel., 22: 263-272 (1992). However, increasing the particle size also has been found to minimize the probability of particles (possessing standard mass density) entering the airways and acini due to excessive deposition in the oropharyngeal or nasal regions. Heyder, J., J. Aerosol Sci., 17: 811-825 (1986).

Local and systemic inhalation therapies can often benefit from a relatively slow controlled release of the therapeutic agent. Gonda, I., "Physico-chemical principles in aerosol delivery," in: Topics in Pharmaceutical Sciences 1991, D.J.A. Crommelin and K.K. Midha, Eds., Stuttgart: Medpharm Scientific Publishers, pp. 95-117 (1992). Slow release from a therapeutic aerosol can prolong the residence of an administered drug in the airways or acini, and diminish the rate of drug appearance in the bloodstream. Also, patient compliance is increased by reducing the frequency of dosing. Langer, R., Science, 249: 1527-1533 (1990); and Gonda, I., "Aerosols for delivery of therapeutic and diagnostic agents to the respiratory tract," in Critical Reviews in Therapeutic Drug Carrier Systems 6: 273-313 (1990).

Controlled release drug delivery to the lung may simplify the way in which many drugs are taken. Gonda, I., Adv. Drug Del. Rev., 5: 1-9 (1990); and Zeng, X., et al., Int. J. Pharm., 124: 149-164 (1995). Pulmonary drug delivery is an attractive alternative to oral, transdermal, and parenteral administration because self-administration is simple, the lungs provide a large mucosal surface for drug absorption, there is no first-pass liver effect of absorbed drugs, and there is reduced enzymatic activity and pH mediated drug degradation compared with the oral route. Relatively high bioavailability of many molecules, including macromolecules, can be achieved via inhalation. Wall, D.A., Drug Delivery, 2: 1-20 1995); Patton, J. and Platz, R., Adv. Drug Del. Rev., 8: 179-196 (1992); and Byron, P., Adv. Drug. Del. Rev., 5: 107-132 (1990). As a result, several aerosol formulations of therapeutic drugs are in use or are being tested for delivery to the lung. Patton, J.S., et al., J Controlled Release, 28: 79-85 (1994); Damms, B. and Bains, W., Nature Biotechnology (1996); Niven, R.W., et al., Pharm. Res., 12(9): 1343-1349 (1995); and Kobayashi, S., et al., Pharm. Res., 13(1): 80-83 (1996).

Drugs currently administered by inhalation come primarily as liquid aerosol formulations. However, many drugs and excipients, especially proteins, peptides (Liu, R., et al., Biotechnol. Bioeng., 37: 177-184 (1991)), and biodegradable carriers such as poly(lactide-co-glycolides) (PLGA), are unstable in aqueous environments for extended periods of time. This can make storage as a liquid formulation problematic. In addition, protein denaturation can occur during aerosolization with liquid formulations. Mumenthaler, M., et al., Pharm. Res., 11: 12-20 (1994). Considering these and other limitations, dry powder formulations (DPF's) are gaining increased interest as aerosol formulations for pulmonary delivery. Damms, B. and W. Bains, Nature Biotechnology (1996); Kobayashi, S., et al., Pharm. Res., 13(1): 80-83 (1996); and Timsina, M., et al., Int. J. Pharm., 101: 1-13 (1994). However, among the disadvantages of DPF's is that powders of ultrafine particulates usually have poor flowability and aerosolization properties, leading to relatively low respirable fractions of aerosol, which are the fractions of inhaled aerosol that escape deposition in the mouth and throat. Gonda, I., in Topics in Pharmaceutical Sciences 1991, D. Crommelin and K. Midha, Editors, Stuttgart: Medpharm Scientific Publishers, 95-117 (1992). A primary concern with many aerosols is particulate aggregation caused by particle-particle interactions, such as hydrophobic, electrostatic, and capillary interactions. An effective dry-powder inhalation therapy for both short and long term release of therapeutics, either for local or systemic delivery, requires a powder that displays minimum aggregation, as well as a means of avoiding or suspending the lung's natural clearance mechanisms until drugs have been effectively delivered.

Particles suitable for delivery to the respiratory system of a patient can be prepared by spray drying from aqueous solutions. A number of proteins, however, denature under aqueous spray drying conditions. In some cases, protein particles which are prepared by spray drying from aqueous solutions tend to be hygroscopic and susceptible to lose their activity at even modest humidity levels.

Spray drying in the presence of polysorbate-20 surfactant has been shown to reduce the aggregation of recombinant growth hormone during spray drying. In another approach, solvents including water and methanol or water and ethanol have been employed to spray dry hollow albumin microcapsules. Neither technique, however, has resulted in both, improved protein stability and reduced protein hygroscopicity.

Therefore, a need exists for methods of producing spray-dried particles which overcome or minimize the above-referenced problems.

EP 0 655 237-A1 discloses a medicinal substance preparation in the form of a suspension aerosol which comprises a spray-dried product which is composed of a medicinal substance, of a surface-active, physiologically tolerated substance which is insoluble in the liquefied propellant, where appropriate a masking flavour and/or a customary auxiliary substance, in a liquefiable, hydrogenated or partially hydrogenated fluorocarbon as propellant. The invention furthermore relates to a process for the production of the medicinal substance preparation which comprises medicinal substances suitable for inhalation and acting locally on the lung for the treatment of airway disorders or asthma.

WO97/26863 discloses a process for the production of a powdered pulmonary surfactant preparation containing a hydrophobic protein serving as a pulmonary surfactant characterised by the fact that an organic solution or suspension containing a hydrophobic protein serving as a pulmonary surfactant and possibly other components is subject to spray drying. Powder preparations are obtained that exhibit very good stability under storage, are easy to reconstitute and which are also suitable for administration by inhalation.

WO 97/44012 discloses microparticles, up to $50\mu$m in size, comprising a therapeutic agent and a surfactant. They can be homogeneously formulated with a HFC propellant.

WO 91/16882 discloses a method for direct spray-drying a solution of lipids and water soluble drug to generate a bulk powder as an alternative to the drying of preformed liposomes. In the present method the lipids are dissolved in a solvent and the water-soluble drug is dissolve in aqueous solvent. The two solutions are combined to form a precipitate-free feed solution which is then spray-dried to generate the bulk powder. Upon rehydration the powder spontaneously forms liposomes having a high drug encapsulation efficiency of approximately 70%. The direct spray-dried powder is particularly useful for drug administration by inhalation.

EP 0 317 120-A1 discloses a novel composition and method for solubilizing amphiphillic drugs in a small amount of organic solvent for use in improved liposomes is disclosed. A phosphatidylglycerol is acidified in a small amount of organic solvent. The amphiphillic drug, such as Amphotericin B, suspended in organic solvent is then added to the acidified phosphatidylglycerol and a soluble complex is formed between the phosphatidylglycerol and the amphiphillic drug. When the liposome composition incorporating the soluble complex is hydrated, the final pH of the hydrating aqueous buffer is carefully controlled. The Amphotericin B liposomes formed have markedly reduced toxicity.

Y.-F. MAA et al: "spray-drying of air-liquid interface sensitive recombinant human growth hormone" Journal of Pharmaceutical Sciences, vol 87, no 2, February 1998 (1998-02), pages 152-159, XP000729419 Washington (US) discloses a study that demonstrated that rhGH degradation (insoluble and soluble aggregate formation), as the consequence of air-liquid interfacial degradation, could be prevented using the appropriate formulation. Adding polysorbate-20 surfactant into the liquid feed (with no presence of sugar protectant) significantly reduced the formation of insoluble protein aggregates, while adding the divalent metal zinc ion effectively suppressed the formation of soluble protein aggregates. The combination of the two yielded a spray-dried rhGH powder having insignificant protein degradation. The authors data suggests that the two components might protect the protein through different mechanisms. Polysorbate molecules occupy the air-liquid interface of spray droplets, thereby reducing the chance for rhGH to form insoluble aggregates by surface denaturation. Two zinc ions associate with two rhGH molecules to form a dimer complex that can resit the formation of soluble protein aggregates. Characterization of spray-dried powders by scanning electron microscopy suggests that both formulation and drying conditions have a strong influence on particle morphology and shape. Overall, spherical rhGH powders of smooth surface and good biochemical quality can be prepared by spray-drying using this formulation with no addition of sugar protectant.

## SUMMARY OF THE INVENTION

The invention relates to methods of producing spray-dried particles, also referred to herein as particles, which have improved protein stability as claimed in claims 1 to 18.

In one embodiment of the invention the method includes combining a protein, a phospholipid and an organic-aqueous co-solvent to form a mixture which is spray-dried to produce spray-dried particles having improved bioactive agent stability. In another embodiment of the invention, the method includes combining a protein, a phospholipid and an organic solvent 10 form a mixture which is spray-dried to produce particles having improved protein stability. In a preferred embodiment, the protein is a therapeutic, prophylactic or a diagnostic agent.

In another embodiment, the phospholipid is selected from the group consisting of phosphatidylcholines, phosphatidylethanolamines, phosphatidylglycerols, phosphatidylserines, phosphatidylinositols and combinations thereof. In yet another embodiment of the invention, the spray dried particles have a tap density less than 0.4 g/cm³.

The invention also relates to the use of an effective amount of the spray-dried particles obtained by the methods of the invention for the manufacture of a medicament for use in the delivery of said medicament to the respiratory tract of a patient in need of treatment, prophylaxis or diagnosis.

The spray dried particles can be used for the manufacture of a medicament for enhanced delivery of a therapeutic, prophylactic or diagnostic agent to the airways or the alveolar region of the lung. The particles may be effectively aerosolized for the manufacture of a medicament for administration to the respiratory tract to permit systemic or local delivery of a wide variety of therapeutic agents. They also optionally may be co-delivered with larger carrier particles, not carrying a therapeutic agent, having, for example, a mean diameter ranging between about 50 $\mu$m and 100 $\mu$m. The particles can be used to form a composition that includes the particles and a pharmaceutically acceptable carrier for administration to a patient, preferably for administration via inhalation.

According to one embodiment of the invention, the spray-dried particles can themselves be used as carriers for the manufacture of a medicament for the delivery of a therapeutic, prophylactic or diagnostic agent to the pulmonary system. According to this embodiment of the invention, a therapeutic, prophylactic or diagnostic agent can be added onto the spray-dried carrier particles for the manufacture of a medicament for delivery to the pulmonary system. Small-sized therapeutic, prophylactic or diagnostic agents, such as, for example, agents having a particle size in the nanometer range, can be carried by the spray-dried carrier particles and delivered to the pulmonary system.

By providing a method for producing spray-dried particles which have increased protein stability, the invention has numerous advantages. In addition, it provides a method for producing aerodynamically light particles suitable for delivery to the respiratory system.

## DETAILED DESCRIPTION OF THE INVENTION

The invention generally relates to methods of producing spray-dried particles which have improved protein stability. The terms "bioactive" and "biologically active" are used herein interchangeably. As used herein the term bioactive agent includes peptides and proteins. Proteins are defined herein as having about 100 amino acid residues or more, while peptides are defined herein as having less than about 100 amino acid residues. As used herein, the term bioactive agent also includes bioactive macromolecules other than peptides or proteins. Examples of such bioactive macromolecules include, but are not limited to: polysaccharides and other sugars, lipids, DNA, RNA, nucleic acid sequences, genes, antisense molecules, antigens and others. In a preferred embodiment of the invention, the protein can be a therapeutic, prophylactic or diagnostic agent.

Specific examples of biologically active agents are but are not limited to: insulin, erythroprotein, interferons, colony stimulating factors, such as, granulocyte colony stimulating factor, growth hormones, such as, for example, human growth hormone, LHRH analogs, LHRH antagonists, tissue plasminogen activator, somatostatin analog, r Factor VIII, r Factor IX, calcitonin, abciximab, dornase alfa, polysaccharides, AG337, bone inducing protein, bone morphogenic protein, brain derived growth factor, gastrin 17 immunogen, interleukins, such as, for example, IL-2, PEF superoxide, infliximab, permeability increasing protein-21, platelet derived growth factor, stem cell factor, Thyrogen[R] and somatomedin C.

As used herein, the term stability generally is related to maintaining the integrity or to minimizing the denaturation, aggregation or unfolding of a biologically active agent such as a protein, peptide or another bioactive macromolecule after being exposed to conditions known to negatively affect its stability. As used herein, improved stability generally means that, under conditions known to result in degradation, denaturation, aggregation or unfolding, the bioactive agent maintains greater stability compared to control particles subjected to the same conditions. Control particles can be, for example, commercially available particles or powders which include the bioactive agent. For example, control particles can include lyophilized bulk proteins or lyophilized sugars. Control particles can also be particles obtained by methods other than the methods of the invention. For example, control particles can include particles that are spray-dried from aqueous solutions or particles that do not include a phospholipid.

Protein degradation, for example, is often facilitated by water. Improved protein stability can be demonstrated in terms of improved retention of protein integrity under storage conditions at specified moisture levels. For example, spray-dried particles having improved protein stability are particles which undergo less degradation, denaturation, aggregation and/or unfolding, relative to protein formulations spray-dried from aqueous solutions, or spray-dried from mixtures that do not include a phospholipid, after storage for six weeks at about 25 °C (e.g. +/-2°C) and about 60% (e.g. +/- 5%) relative humidity. If more severe conditions are employed, spray-dried particles having improved protein stability are particles which, after storage for six weeks at about 40°C (e.g. +/- 2°C) and about 75% (e.g. +/- 5%) relative humidity, retain greater protein stability (or undergo less degradation, denaturation, aggregation and/or unfolding) compared to protein formulations spray-dried from aqueous solutions or spray-dried from mixtures that do not include a phospholipid. In one embodiment of the invention the spray-dried particles retain at least about 70%, preferably at least about 80% protein integrity, when stored at about 25 °C and about 60% relative humidity conditions for six weeks. In another embodiment of the invention the spray-dried particles retain at least about 50%, preferably at least about 60% protein integrity when stored at about 40°C and about 75% relative humidity conditions for six weeks.

Bioactive agent stability or integrity can be measured by techniques such as those known in the art. For example, protein stability can be measured by size exclusion high performance liquid chromatography (SEC HPLC). Other suitable techniques for detecting bioactive agent stability, aggregation or degradation include, but are not limited to: reverse phase high performance liquid chromatography (RP HPLC); sodium dodecyl sulfate polyacrylamide gel electrophoresis (SDS PAGE); enzyme-linked immunoadsorbent assay (ELISA) and radioimmunoassay (RIA).

In one embodiment, the method for producing spray-dried particles having improved bioactive agent stability includes combining a bioactve agent, such as, for example, the agents described above, with a phospholipid and a co-solvent to form a mixture.

Co-solvents include an aqueous solvent and an organic solvent. Suitable organic solvents that can be employed include but are not limited to alcohols such as, for example, ethanol, methanol, propanol, isopropanol and butanols. Other organic solvents include but are not limited to perfluorocarbons, dichloromethane, chloroform, ether, ethyl acetate, methyl tert-butyl ether and others. Aqueous solvents include water and buffered solutions. In a preferred embodiment, the organic solvent is ethanol. Preferably, the amount of organic solvent can be present in the co-solvent in an amount ranging from about 50 to about 90% by volume. In a more preferred embodiment, the organic solvent is present in the co-sol vent in an amount ranging from about 60 to about 85% by volume.

In another embodiment, the method for producing spray-dried particles having improved bioactive agent stability includes combining a bioactive agent, such as, for example, the agents described above, with a phospholipid and an organic solvent to form a mixture. The organic solvent includes but is not limited to the organic solvents described above.

In a preferred embodiment of the invention, the phospholipid, also referred to herein as phosphoglyceride, is a phospholipid endogenous to the lung. Such a phospholipid is particularly advantageous in preparing spray-dried particles suitable for delivery to the respiratory system of a patient.

In another preferred embodiment the phospholipid is selected from the group consisting of phosphatidylcholines, phosphatidylethanolamines, phosphatidylglycerols, phosphatidylserines, phosphatidylinositols and combinations thereof. Specific examples of phospholipids include but are not limited to phosphatidylcholines dipalmitoyl phosphatidylcholine (DPPC), dipalmitoyl phosphatidylethanolamine (DPPE), distearoyl phosphatidylcholine (DSPC), dipalmitoyl phosphatidyl glycerol (DPPG) or any combinations thereof.

The mixture can have a neutral, acidic or alkaline pH. Optionally, a pH buffer can be added to the solvent or co-solvent or to the formed mixture. Preferably, the pH can range from about 3 to about 10.

The mixture obtained by combining the bioactive agent with the phospholipid and the co-solvent is spray-dried. Suitable spray-drying techniques are described, for example, by K. Masters in "Spray Drying Handbook", John Wiley & Sons, New York, 1984. Generally, during spray-drying, heat from a hot gas such as heated air or nitrogen is used to evaporate the solvent from droplets formed by atomizing a continuous liquid feed.

In a preferred embodiment, a rotary atomizer is employed. Examples of suitable spray driers using rotary atomization include Niro spray drier Mobile Minor. According to the invention, the phospholipid, is present in the spray-dried particles in an amount of at least about 10 weight %. The amount of phospholipid to be included in the particles can be determined experimentally by determining the amount of phospholipid which, when included in the spray-dried particles, results in improved stability, measured by means such as, but not limited to, those described above.

In one embodiment, the spray-dried particles include a protein, which is present in the particles in an amount ranging from 30 to 90 weight %.

Without being held to any particular mechanism, it is believed that the improved stability is at least in part the result of a lowered tendency of the protein to be situated at the air-water interface or air-co-solvent interface of the droplet. The phospholipid is believed to compete with the protein for the air-droplet interface, thereby protecting the protein. Furthermore, it is believed that the presence of the phospholipid also renders the spray-dried particles less prone to degradation owing to exposure to high humidity conditions during storage.

According to the invention, the spray-dried particles consist of and a phospholipid, such as, for example, the phospholipids described above.

According to another embodiment of the invention, the particles consist essentially of bioactive agent and phospholipid. For example, the spray-dried particles can further include small or trace amounts of residual solvent or co-solvent, impurities, substances which control the pH, or other materials in small or trace amounts. Ranges for impurity levels and for residual solvent levels are generally well established in the industry and known to those skilled in the art. Amounts of pH buffers that can be added to the solvent, co-solvent or mixture are also known in the art.

In a preferred embodiment, the spray-dried particles have a tap density less than about 0.4 g/cm$^3$. In another embodiment, the spray-dried particles have a tap density less than about 0.1 g/cm$^3$. In yet another embodiment, the spray-dried particles have a tap density less than about 0.05 g/cm$^3$.

As used herein, the phrase "aerodynamically light particles" refers to particles having a tap density less than about 0.4 g/cm$^3$. The tap density of particles of a dry powder may be obtained using a GeoPyc™ instrument (Micrometrics Instrument Corp., Norcross, GA 30093). A Dual Platform Microprocessor Controlled Tap Density Tester (Vankel, NC) can also be used. Tap density is a standard measure of the envelope mass density. The envelope mass density of an isotropic particle is defined as the mass of the particle divided by the minimum sphere envelope volume within which it can be enclosed. Features which can contribute to low tap density include irregular surface texture and porous structure.

The preferred median diameter for aerodynamically light particles for inhalation therapy is at least about 5 microns ($\mu$m), for example between about 5 and about 30 $\mu$m. In a preferred embodiment, the spray-dried particles have a median geometric diameter of between about 5 $\mu$m and about 30 $\mu$m. Terms such as median diameter, mass median diameter (MMD), mass median geometric diameter (MMGD) and mass median envelope diameter (MMED) are herein used interchangeably. The term diameter, in contrast with the term "aerodynamic diameter", refers herein to mass or geometric diameter. The terms "aerodynamic diameter" and "mass median aerodynamic diameter" (MMAD) are used herein interchangeably. In one embodiment of the invention, the mass median aerodynamic diameter is between about 1 $\mu$m and about 5 $\mu$m. In another embodiment of the invention, the mass median aerodynamic diameter is between about 1 $\mu$m and about 3 $\mu$m. In another embodiment, the mass median aerodynamic diameter is between about 3 $\mu$m and about 5 $\mu$m.

The mass median diameter of the spray-dried particles can be measured using an electrical zone sensing instrument such as Coulter Multisizer IIe (Coulter, Miami, FL) or a laser diffraction instrument (for example a Helos instrument manufactured by Sympatec, Princeton, NJ). The diameter of particles in a sample will range depending upon factors such as particle composition and methods of synthesis. The distribution of size of particles in a sample can be selected to permit optimal deposition within targeted sites within the respiratory tract.

Aerodynamically light particles may be fabricated or separated, for example by filtration or centrifugation, to provide a particle sample with a preselected size distribution. For example, greater than 30%, 50%, 70%, or 80% of the particles in a sample can have a diameter within a selected range of at least about 5 $\mu$m. The selected range within which a certain percentage of the particles must fall may be for example, between about 5 and about 30 $\mu$m, or optionally between about 5 and about 15 $\mu$m. In one preferred embodiment, at least a portion of the particles have a diameter between about 9 and about 11 $\mu$m. Optionally, the particle sample also can be fabricated wherein at least about 90%, or optionally about 95% or about 99%, have a diameter within the selected range. The presence of the higher proportion of the aerodynamically light, larger diameter particles in the particle sample enhances the delivery of therapeutic or diagnostic agents incorporated therein to the deep lung. Large diameter particles generally mean particles having a median geometric diameter of at least about 5 $\mu$m.

Aerodynamically light particles with a tap density less than about 0.4 g/cm$^3$, median diameters of at least about 5 $\mu$m, and an aerodynamic diameter of between about one and about five microns, preferably between about one and about three microns, are more capable of escaping inertial and gravitational deposition in the oropharyngeal region, and are targeted to the airways or the deep lung. The use of larger, more porous particles is advantageous since they are able to aerosolize more efficiently than smaller, denser aerosol particles such as those currently used for inhalation therapies.

In comparison to smaller, relatively denser particles the larger aerodynamically light particles, preferably having a median diameter of at least about 5 $\mu$m, also can potentially more successfully avoid phagocytic engulfment by alveolar macrophages and clearance from the lungs, due to size exclusion of the particles from the phagocytes' cytosolic space. Phagocytosis of particles by alveolar macrophages diminishes precipitously as particle diameter increases beyond about 3$\mu$ m. Kawaguchi, H., et al., Biomaterials 7: 61-66 (1986); Krenis, L.J. and Strauss, B., Proc. Soc. Exp. Med., 107: 748-750 (1961); and Rudt, S. and Muller, R.H., J. Contr. Rel., 22: 263-272 (1992). For particles of statistically isotropic shape, such as spheres with rough surfaces, the particle envelope volume is approximately equivalent to the volume of cytosolic space required within a macrophage for complete particle phagocytosis.

Aerodynamically light particles thus are capable of a longer term release of an encapsulated agent in the lungs. Following inhalation, aerodynamically light biodegradable particles can deposit in the lungs, and subsequently undergo slow degradation and drug release, without the majority of the particles being phagocytosed by alveolar macrophages. The drug can be delivered relatively slowly into the alveolar fluid, and at a controlled rate into the blood stream, minimizing possible toxic responses of exposed cells to an excessively high concentration of the drug. The aerodynamically light particles thus are highly suitable for inhalation therapies, particularly in controlled release applications.

The particles may be fabricated with the appropriate material, surface roughness, diameter and tap density for localized delivery to selected regions of the respiratory tract such as the deep lung or upper airways. For example, higher density or larger particles may be used for upper airway delivery, or a mixture of varying sized particles in a sample, provided with the same or different therapeutic agent may be administered to target different regions of the lung in one administration. Particles having an aerodynamic diameter in the range from about 3 to about 5 $\mu$m are preferred for delivery to the central and upper airways. Particles having an aerodynamic diameter in the range from about 1 to about 3 $\mu$m are preferred for delivery to the deep lung.

Inertial impaction and gravitational settling of aerosols are predominant deposition mechanisms in the airways and acini of the lungs during normal breathing conditions. Edwards, D.A., J. Aerosol Sci., 26: 293-317 (1995). The importance of both deposition mechanisms increases in proportion to the mass of aerosols and not to particle (or envelope) volume. Since the site of aerosol deposition in the lungs is determined by the mass of the aerosol (at least for particles of mean aerodynamic diameter greater than approximately 1 $\mu$m), diminishing the tap density by increasing particle surface irregularities and particle porosity permits the delivery of larger particle envelope volumes into the lungs, all other physical parameters being equal.

The low tap density particles have a small aerodynamic diameter in comparison to the actual envelope sphere diameter. The aerodynamic diameter, $d_{aer}$, is related to the envelope sphere diameter, $d$ (Gonda, I., "Physico-chemical principles in aerosol delivery," in Topics in Pharmaceutical Sciences 1991 (eds. D.J.A. Crommelin and K.K. Midha), pp. 95-117, Stuttgart: Medpharm Scientific Publishers, 1992)), by the formula:

$$d_{aer} = d\sqrt{\rho}$$

.

where the envelope mass p is in units of g/cm³. Maximal deposition of monodisperse aerosol particles in the alveolar region of the human lung (~60%) occurs for an aerodynamic diameter of approximately $d_{aer}$=3 $\mu$m. Heyder, J. et al., J. Aerosol Sci., 17: 811-825 (1986). Due to their small envelope mass density, the actual diameter d of aerodynamically light particles comprising a monodisperse inhaled powder that will exhibit maximum deep-lung deposition is:

$$d = 3/\sqrt{\rho} \ \mu m \ (\text{where } \rho < 1 \ g/cm^3);$$

where d is always greater than 3 $\mu$m. For example, aerodynamically light particles that display an envelope mass density, p = 0.1 g/cm³, will exhibit a maximum deposition for particles having envelope diameters as large as 9.5 $\mu$m. The increased particle size diminishes interparticle adhesion forces. Visser, J., Powder Technology, 58: 1-10. Thus, large particle size increases efficiency of aerosolization to the deep lung for particles of low envelope mass density, in addition to contributing to lower phagocytic losses.

In one embodiment of the invention, the spray-dried particles have a tap density less than about 0.4 g/cm³ and a median diameter between about 5 $\mu$m and about 30 $\mu$m, which in combination yield an aerodynamic diameter of between about 1 and about 5 $\mu$m, preferably between about 1 and about 3 $\mu$m. The aerodyanamic diameter is calculated to provide for maximum deposition within the lungs, previously achieved by the use of very small particles of less than five microns in diameter, preferably between one and three microns, which are then subject to phagocytosis. Selection of particles which have a larger diameter, but which are sufficiently light (hence the characterization "aerodynamically light"), results in an equivalent delivery to the lungs, but the larger size particles are not phagocytosed. Improved delivery can be obtained by using particles with a rough or uneven surface relative to those with a smooth surface.

According to one embodiment of the invention, the particles have a mass density of less than about 0.4 g/cm$^3$ and a mean diameter of between about 5 $\mu$m and about 30 $\mu$m. Mass density and the relationship between mass density, mean diameter and aerodynamic diameter are discussed in U. S. Application No. 08/655,570, filed on May 24,1996. In a preferred embodiment, the aerodynamic diameter of particles having a mass density less than about 0.4 g/cm$^3$ and a mean diameter of between about 5 $\mu$m and about 30 $\mu$m is between about 1 $\mu$m and about 5 $\mu$m.

The spray-dried particles can be used for the manufacture of a medicament for controlled systemic or local delivery of therapeutic or diagnostic agents to the respiratory tract via aerosolization. Administration of the particles to the lung by aerosolization permits deep lung delivery of relatively large diameter therapeutic aerosols, for example, greater than about 5 $\mu$m in median diameter. The particles can be fabricated with a rough surface texture to reduce particle agglomeration and improve flowability of the powder. The spray-dried particles have improved aerosolization properties. The spray-dried particle can be fabricated with features which enhance aerosolization via dry powder inhaler devices, and lead to lower deposition in the mouth, throat and inhaler device.

Aerosol dosage, formulations and delivery systems may be selected for a particular therapeutic application, as described, for example, in Gonda, I. "Aerosols for delivery of therapeutic and diagnostic agents to the respiratory tract," in Critical Reviews in Therapeutic Drug Carrier Systems, 6: 273-313, 1990; and in Moren, "Aerosol dosage forms and formulations," in: Aerosols in Medicine. Principles, Diagnosis and Therapy, Moren, et al., Eds, Esevier, Amsterdam, 1985.

The greater efficiency of aerosolization by the particles disclosed herein relative to particles that do not include a surfactant or a charged complex of a therapeutic agent permits more of a therapeutic agent to be delivered. The use of biodegradable polymers permits controlled release in the lungs and long-time local action or systemic bioavailability. Denaturation of macromolecular drugs can be minimized during aerosolization since macromolecules can be contained and protected within a polymeric shell. Coencapsulation of peptides with peptidase-inhibitors can minimize peptide enzymatic degradation. Pulmonary delivery advantageously can reduce or eliminate the need for injection. For example, the requirement for daily insulin injections can be avoided.

The invention is also related to a method for delivery to the pulmonary system. The method comprises administering to the respiratory tract of a patient in need of treatment, prophylaxis or diagnosis an effective amount of the spray dried particles obtained by the methods of the invention.

Porous or aerodynamically light particles, having a geometric size (or mean diameter) in the range of about 5 to about30 micrometers, and tap density less than about 0.4 g/cm$^3$, such that they possess an aerodynamic diameter of about 1 to about 3 $\mu$m, have been shown to display ideal properties for delivery to the deep lung. Larger aerodynamic diameters, preferably ranging, for example from about 3 to about 5 $\mu$m are preferred, however, for delivery to the central and upper airways. According to one embodiment of the invention the particles have a tap density of less than about 0.4 g/cm$^3$ and a mean diameter of between about 5 $\mu$m and about 30 $\mu$m. According to another embodiment of the invention, the non-polymeric particles have a mass density of less than about 0.4 g/cm$^3$ and a mean diameter of between about 5 $\mu$m and about 30 $\mu$m. In one embodiment of the invention, the particles have an aerodynamic diameter between about one and about five microns. In another embodiment of the invention, the particles have an aerodynamic diameter between about one and about three microns. In still another embodiment of the invention, the particles have an aerodynamic diameter between about three and about five microns.

For therapeutic, diagnosis or prophylactic use, particles can be delivered from an inhaler device, such as, but not limited to a metered-dose-inhaler (MDI), dry-powder inhaler (DPI) nebulizer or by instillation. Such devices are known in the art. For example, a DPI is described in U.S. Patent No. 4,069,819 issued to Valentini, et al. on August 5,1976.

EXEMPLIFICATIONS

The present invention will be further understood by reference to the following non-limiting examples.

Some of the methods and materials employed in the following examples are described in U.S. Application No. 09/211,940, filed December 15, 1998, in U.S. Application No. 08/739,308, filed October 29,1996, now U.S. Patent No. 5,874,064, in U.S. Application No. 08/655,570, filed May 24, 1996, in U.S. Application No. 09/194,068, filed May 23, 1997, in PCT/US97/08895 application filed May 23, 1997, in U.S. Application 08/971,791, filed November 17,1997, in U.S. application No. 08/784,421, filed January 16,1997, now U.S. Patent No. 5,855,913 and in U.S. Application No. 09/337,245, filed on June 22, 1999,

Materials

IgG was obtained from Sigma, St. Louis, MO. The DNA sequence of hGH is described in U.S. patent No, 4,898,830 issued on February 6, 1990 to Goeddel et al. DPPC was obtained from Avanti (Alabaster, ALA) or Sigma.

Spray Drying

A Mobile Minor spray-drier from Niro (Denmark) was used. The hot gas was dehumidified air or nitrogen. The gas temperature ranged from about 80 to about 150°C.

Particle Size Distribution Analysis

Size distributions were determined using a Coulter Multisizer II (Coulter Corp., Miami, FL). Approximately 10 drops Coulter type IA non-ionic dispersant were added, followed by 2 mL isoton II solution (Coulter), to 5-10 mg microspheres, and the particles were dispersed by brief vortex mixing. This suspension was added to 50 mL isoton II solution until the coincidence of particles was between 5 and 8 %. Greater than 500,000 particles were counted for each batch of spheres.

Particle Morphology by Scanning Electron Microscopy (SEM)

Microsphere morphology was observed by scanning electron microscopy (SEM) using a Stereoscan 250 MK3 microscope from Cambridge Instruments (Cambridge, MA) at 15 kV. Microspheres were freeze-dried, mounted on metal stubs with double-sided tape, and coated with gold prior to observation.

Particle Density Analysis

Bulk density was estimated by tap density measurements, such as obtained using a Dual Platform Microprocessor Controlled Tap Density Tester (Vankel, NC) and confirmed by mercury intrusion analysis at Porous Materials, Inc. (Ithaca, NY).

Protein Integrity Measurements

Protein integrity measurements were obtained by size exclusion chromatography (SEC-HPLC).

Example 1

IgG particles were prepared to demonstrate that particles suitable for inhalation, with theoretical aerodynamic diameter between 1 and 3 microns, could be prepared by spray drying a protein with DPPC in a water/ethanol cosolvent mixture. A 70/30 ethanol/water co-solvent was employed. The solute concentration (combined IgG and DPPC) was 0.1 % w/v. The pH of the water varied from 3-5.7. The spray drying parameters were inlet temperature (110°C), atomizer spin rate (1-2 bar), feed rate of 40 mL/min, and outlet temperature near 50°C. 40/60 IgG/DPPC and 50/50 IgG/DPPC particles were prepared. The tap densities ranged from 0.02 to 0.2 g/cm$^3$ with mean geometric diameters near 7 microns. This gave aerodynamic diameters in the range of 1-3 microns, ideal for inhalation. SDS PAGE analysis showed no difference between starting IgG material and spray dried IgG particle indicating no aggregates or degradation.

Example 2

Human growth hormone (hGH) is one example of a protein susceptible to denaturation during spray drying from an aqueous solution. hGH was spray-dried in an aqueous/ethanol co-solvent mixture with DPPC.

A 70/30 ethanol/aqueous co-solvent (vol/vol) and solute concentrations (combined hGH and DPPC) of 0.1% w/v were used. The pH was 7.4 (NaPO$_4$ buffer). The spray drying parameters were as described above. 60/40 hGH/ DPPC and 80/20 hGH/DPPC particles were prepared.

The tap densities ranged from 0.02 to 0.04 g/cc with mean geometric diameters of 7-8 microns. This gave aerodynamic diameters in the range of 1-3 microns, ideal for inhalation.

The 60% and 80% hGH spray dried particles were analyzed by HPLC to detect instabilities (e.g. aggregation, deamidation). The spray dried particles described above were compared to hGH particles that were spray dried without the presence of DPPC and an organic co-solvent. The analysis showed a reduction of aggregates from 23% to less than 2%, and no detectable deamidation.

Example 3

The stability of hGH protein over an extended period of time (6 weeks) upon exposure to different temperatures and humidities was determined. These experiments were carried out in loosely capped glass vials providing samples to full exposure to temperature and humidity. The conditions selected were harsher than those generally present during product storage.

The stability of hGH was measured by SEC-HPLC which shows the amount of monomer (the active form) and higher molecular weight aggregates. Table 1 presents the SEC-HPLC data demonstrating the better stability of the hGH spray-dried particles (60% hGH and 40% DPPC) versus bulk hGH powder.

Table 1

| Stability Condition | % Monomer hGH/DPPC Particles (6 weeks) | % Monomer bulk hGH Powder (6 weeks) |
|---|---|---|
| 25±2°C & 60±5% RH | 88.9 | 25.4 |
| 40±2°C & 75±5% RH | 68.4 | 37.1 |

**Claims**

1. A method for producing spray-dried particles having improved stability of a protein comprising:

   (a) combining a protein having 100 amino acids or more, a phospholipid and an organic solvent, to form a mixture; and
   (b) spray-drying said mixture to produce spray-dried particles having improved stability of the protein;

   wherein the spray-dried particles consist of the protein in an amount from 30 to 90% by weight, phospholipid in an amount of at least 10% by weight, optional buffer and trace amounts of: residual solvent or co-solvent, impurities, or other materials in trace amounts.

2. The method of claim 1, wherein the phospholipid is selected from the group consisting of phosphatidylcholines, phosphatidylethanolamines, phosphatidylglycerols, phosphatidylserines, phosphatidylinositols and combinations thereof.

3. The method of claim 1, wherein the protein is human growth hormone.

4. The method of claim 1, wherein the spray-dried particles retain at least 70% protein integrity when stored at 25°C and 60% relative humidity conditions for six weeks.

5. The method of claim 1, wherein the spray-dried particles retain at least 50% protein integrity when stored at 40°C and 75% relative humidity conditions for six weeks.

6. The method of claim 1, wherein the protein is a therapeutic, prophylactic or diagnostic agent.

7. The method of claim 1, wherein the protein and phospholipid concentration in said mixture is at least 0.1 weight/volume%.

8. The method of claim 1, wherein the organic solvent includes an alcohol;

9. The method of claim 1, wherein the organic solvent is a co-solvent in a concentration of at least 50 volume %.

10. The method of claim 1, wherein the spray-dried particles have a tap density less than 0.4g/cm$^3$,

11. The method of claim 10, wherein the spray-dried particles have a tap density of less than 0.1 g/cm$^3$.

12. The method of claim 11, wherein the spray-dried particles have a tap density of less than 0.05 g/cm$^3$.

13. The method of claim 10, wherein the spray-dried particles have a median geometric diameter of between 5 microns and 30 microns.

14. The method of claim 13, wherein the spray-dried particles have an aerodynamic diameter of between 1 micron and 5 microns.

15. The method according to any one of claims 1 to 14, wherein the organic solvent is part of a co-solvent which co-solvent also includes an aqueous solvent.

16. The method according to any one of claims 1 to 15, wherein the spray-dried particles consist of the protein, the phospholipid and buffer.

17. The method according to any one of the preceding claims, wherein the phospholipid is endogenous to the lung.

18. The method according to any one of the preceding claims, wherein the particles are in the form of a dry powder for administration using a dry powder inhaler to the respiratory tract.

19. Particles producible by the method according to any one of claims 1 to 18.

20. The particles of claim 19 for use in therapy.

21. Use of the particles of claim 20 for the manufacture of a medicament for use in the delivery of said medicament to the respiratory tract.


**Patentansprüche**

1. Ein Verfahren zur Herstellung sprühgetrockneter Partikel mit verbesserter Stabilität eines Proteins, umfassend:

   (a) Vereinigen eines Proteins mit 100 Aminosäuren oder mehr, eines Phospholipids und eines organischen Lösemittels, um ein Gemisch zu bilden; und
   (b) Sprühtrocknen besagten Gemisches, um sprühgetrocknete Partikel mit verbesserter Stabilität des Proteins herzustellen;

   wobei die sprühgetrockneten Partikel aus dem Protein in einer Menge von 30 bis 90 Gew.-%, Phospholipid in einer Menge von wenigstens 10 Gew.-%, wahlweise Puffer und Spurenmengen von: Lösemittel- oder Co-Lösemittelrückständen, Verunreinigungen oder anderen Stoffen in Spurenmengen bestehen.

2. Das Verfahren nach Anspruch 1, worin das Phospholipid aus der Gruppe ausgewählt ist, bestehend aus Phosphatidylcholinen, Phosphatidylethanolaminen, Phosphatidylglycerolen, Phosphatidylserinen, Phosphatidylinositolen und Kombinationen davon.

3. Das Verfahren nach Anspruch 1, worin das Protein humanes Wachstumshormon ist.

4. Das Verfahren nach Anspruch 1, worin die sprühgetrockneten Partikel wenigstens 70% Proteinintegrität beibehalten, wenn sie unter Bedingungen von 25°C und 60% relativer Luftfeuchtigkeit 6 Wochen gelagert sind.

5. Das Verfahren nach Anspruch 1, worin die sprühgetrockneten Partikel wenigstens 50% Proteinintegrität beibehalten, wenn sie unter Bedingungen von 40°C und 75% relativer Luftfeuchtigkeit 6 Wochen gelagert sind.

6. Das Verfahren nach Anspruch 1, worin das Protein ein therapeutisches, prophylaktisches oder diagnostisches Agens ist.

7. Das Verfahren nach Anspruch 1, worin die Protein- und Phospholipidkonzentration in besagtem Gemisch wenigstens 0,1 Gew./Vol.-% beträgt.

8. Das Verfahren nach Anspruch 1, worin das organische Lösemittel einen Alkohol umfasst.

9. Das Verfahren nach Anspruch 1, worin das organische Lösemittel ein Co-Lösemittel in einer Konzentration von wenigstens 50 Vol.-% ist.

**10.** Das Verfahren nach Anspruch 1, worin die sprühgetrockneten Partikel eine Klopfdichte von weniger als 0,4 g/cm$^3$ besitzen.

**11.** Das Verfahren nach Anspruch 10, worin die sprühgetrockneten Partikel eine Klopfdichte von weniger als 0,1 g/cm$^3$ besitzen.

**12.** Das Verfahren nach Anspruch 11, worin die sprühgetrockneten Partikel eine Klopfdichte von weniger als 0,05 g/cm$^3$ besitzen.

**13.** Das Verfahren nach Anspruch 10, worin die sprühgetrockneten Partikel einen mittleren geometrischen Durchmesser zwischen 5 μm und 30 μm besitzen.

**14.** Das Verfahren nach Anspruch 13, worin die sprühgetrockneten Partikel einen aerodynamischen Durchmesser zwischen 1 μm und 5 μm besitzen.

**15.** Das Verfahren gemäß einem der Ansprüche 1 bis 14, worin das organische Lösemittel Teil eines Co-Lösemittels ist, wobei dieses Co-Lösemittel auch ein wässriges Lösemittel umfasst.

**16.** Das Verfahren gemäß einem der Ansprüche 1 bis 15, worin die sprühgetrockneten Partikel aus dem Protein, dem Phospholipid und Puffer bestehen.

**17.** Das Verfahren gemäß einem der vorhergehenden Ansprüche, worin das Phospholipid für die Lunge endogen ist.

**18.** Das Verfahren gemäß einem der vorhergehenden Ansprüche, worin die Partikel in Form eines trockenen Pulvers vorliegen, geeignet zur Verabreichung mit Hilfe eines Trockenpulver-Inhalators für den Respirationstrakt.

**19.** Partikel, die mit dem Verfahren gemäß einem der Ansprüche 1 bis 18 herstellbar sind.

**20.** Die Partikel nach Anspruch 19 zur therapeutischen Anwendung.

**21.** Verwendung der Partikel nach Anspruch 20 für die Herstellung eines Medikamentes zur Anwendung bei der Abgabe besagten Medikamentes an den Respirationstrakt.


**Revendications**

**1.** Procédé de production de particules d'une protéine séchées par atomisation ayant une stabilité améliorée comprenant :

(a) la combinaison d'une protéine ayant 100 acides aminés ou plus, d'un phospholipide et d'un solvant organique, pour former un mélange ; et
(b) le séchage par atomisation dudit mélange pour produire des particules de la protéine séchées par atomisation ayant une stabilité améliorée ;

dans lequel les particules séchées par atomisation sont constituées de la protéine en une quantité de 30 à 90 % en poids, du phospholipide en une quantité d'au moins 10 % en poids, d'un éventuel tampon et de quantités traces : d'un solvant ou d'un cosolvant résiduel, d'impuretés ou d'autres matériaux dans des quantités traces.

**2.** Procédé selon la revendication 1, dans lequel le phospholipide est choisi dans le groupe constitué de phosphatidylcholines, phosphatidyléthanolamines, phosphatidylglycérols, phosphatidylsérines, phosphatidylinositols et de combinaisons de ceux-ci.

**3.** Procédé selon la revendication 1, dans lequel la protéine est l'hormone de croissance humaine.

**4.** Procédé selon la revendication 1, dans lequel les particules séchées par atomisation conservent au moins 70 % de l'intégrité de la protéine lorsqu'elles sont stockées à 25°C et dans des conditions d'humidité relative à 60 % pendant six semaines.

**5.** Procédé selon la revendication 1, dans lequel les particules séchées par atomisation conservent au moins 50 % de l'intégrité de la protéine lorsqu'elles sont stockées à 40°C et dans des conditions d'humidité relative à 75 % pendant six semaines.

**6.** Procédé selon la revendication 1, dans lequel la protéine est un agent thérapeutique, prophylactique ou diagnostique.

**7.** Procédé selon la revendication 1, dans lequel la concentration de la protéine et du phospholipide dans ledit mélange est d'au moins 0,1 % en poids/volume.

**8.** Procédé selon la revendication 1, dans lequel le solvant organique comprend un alcool.

**9.** Procédé selon la revendication 1, dans lequel le solvant organique est un cosolvant dans une concentration d'au moins 50 % en volume.

**10.** Procédé selon la revendication 1, dans lequel les particules séchées par atomisation ont une densité tapée inférieure à 0,4 g/cm$^3$.

**11.** Procédé selon la revendication 10, dans lequel les particules séchées par atomisation ont une densité tapée inférieure à 0,1 g/cm$^3$.

**12.** Procédé selon la revendication 11, dans lequel les particules séchées par atomisation ont une densité tapée inférieure à 0,05 g/cm$^3$.

**13.** Procédé selon la revendication 10, dans lequel les particules séchées par atomisation ont un diamètre géométrique médian entre 5 microns et 30 microns.

**14.** Procédé selon la revendication 13, dans lequel les particules séchées par atomisation ont un diamètre aérodynamique entre 1 micron et 5 microns.

**15.** Procédé selon l'une quelconque des revendications 1 à 14, dans lequel le solvant organique fait partie d'un cosolvant, lequel cosolvant comprend également un solvant aqueux.

**16.** Procédé selon l'une quelconque des revendications 1 à 15, dans lequel les particules séchées par atomisation sont constituées de la protéine, du phospholipide et de tampon.

**17.** Procédé selon l'une quelconque des revendications précédentes, dans lequel le phospholipide est endogène au poumon.

**18.** Procédé selon l'une quelconque des revendications précédentes, dans lequel les particules sont sous la forme d'une poudre sèche destinée à une administration aux voies respiratoires à l'aide d'un inhalateur de poudre sèche.

**19.** Particules susceptibles d'être produites par le procédé selon l'une quelconque des revendications 1 à 18.

**20.** Particules selon la revendication 19 destinées à une utilisation en thérapie.

**21.** Utilisation des particules selon la revendication 20 pour la fabrication d'un médicament destiné à une utilisation dans la délivrance dudit médicament aux voies respiratoires.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0655237 A1
- WO 9726863 A
- WO 9744012 A
- WO 9116882 A
- EP 0317120 A1
- US 65557096 A
- US 4069819 A, Valentini
- US 21194098 A
- US 73930896 A
- US 5874064 A
- US 19406897 A
- US 9708895 W
- US 97179197 A
- US 78442197 A
- US 5855913 A
- US 337245 A
- US 4898830 A, Goeddel

**Non-patent literature cited in the description**

- **ADJEI, A. ; GARREN, J.** *Pharm. Res,* 1990, vol. 7, 565-569
- **ZANEN, P ; LAMM, J.-W.J.** *Int. J. Pharm,* 1995, vol. 114, 111-115
- Aerosols for delivery of therapeutic and diagnostic agents to the respiratory tract. *Critical Reviews in Therapeutic Drug Carrier Systems,* 1990, vol. 6, 273-313
- **ANDERSON.** *Am. Rev. Respir. Dis,* 1989, vol. 140, 1317-1324
- **PATTON ; PLATZ.** *Advanced Drug Delivery Reviews,* 1992, vol. 8, 179-196
- **TIMSINA.** *nt. J. Pharm.,* 1995, vol. 101, 1-13
- **TANSEY, LP.** *Spray Technol. Market,* 1994, vol. 4, 26-29
- **FRENCH, D.L ; EDWARDS, D.A ; NIVEN, R.W.** *J. Aerosol Sci,* 1996, vol. 27, 769-783
- **VISSER, J.** *Powder Technology,* 1989, vol. 58, 1-10
- **RUDT, S ; R.H. MULLER.** *J. Controlled Release,* 1992, vol. 22, 263-272
- **TABATA, Y ; Y. IKADA.** *. Biomed Mater. Res.,* 1988, vol. 22, 837-858
- **GANDERTON, D.** *J Biopharmaceutical Sciences,* 1992, vol. 3, 101-105
- Physico-Chemical Principles in Aerosol Delivery. **GONDA, I.** Topics in Pharmaceutical Sciences. Medpharm Scientific Publishers, 1991, 95-115
- **FRENCH, D.L ; EDWARDS, D.A ; NIVEN, R.W.** *J. Aerosol Sci.,* 1996, vol. 27, 769-783
- Lung Mucociliary Clearance. **PAVIA, D.** Aerosols and the Lung: Clinical and Experimental Aspects. Butterworths, 1984
- **ANDERSON.** *Am. Rev. Respir. Dis.,* 1989, vol. 140, 1317-1324
- **WARHEIT, M.B ; HARTSKY, M.A.** *Microscopy Res. Tech,* 1993, vol. 26, 412-422
- Physiology and Pathophysiology of Pulmonary Macrophages. **BRAIN, J.D.** The Reticuloendothelial System. Plenum, 1985, 315-327
- **DORRIES, A.M ; VALBERG, P.A.** *Am. Rev. Resp. Disease,* 1991, vol. 146, 831-837
- **GEHR, P.** *Microscopy Res. and Tech,* 1993, vol. 26, 423-436
- **KAWAGUCHI, H.** *Biomaterials,* 1986, vol. 7, 61-66
- **KRENIS, L.J ; STRAUSS, B.** *Proc. Soc. Exp. Med,* 1961, vol. 107, 748-750
- **RUDT, S ; MULLER, R.H.** *J Contr. Rel,* 1992, vol. 22, 263-272
- **HEYDER, J.** *J. Aerosol Sci,* 1986, vol. 17, 811-825
- Physico-chemical principles in aerosol delivery. **GONDA, I.** Topics in Pharmaceutical Sciences. Medpharm Scientific Publishers, 1991, 95-117
- **LANGER, R.** *Science,* 1990, vol. 249, 1527-1533
- **GONDA, I.** Aerosols for delivery of therapeutic and diagnostic agents to the respiratory tract. *Critical Reviews in Therapeutic Drug Carrier Systems,* 1990, vol. 6, 273-313
- **GONDA, I.** *Adv. Drug Del. Rev.,* 1990, vol. 5, 1-9
- **ZENG, X et al.** *Int. J. Pharm,* 1995, vol. 124, 149-164
- **WALL, D.A.** *Drug Delivery,* 1995, vol. 2, 1-20
- **PATTON, J ; PLATZ, R.** *Adv. Drug Del. Rev.,* 1992, vol. 8, 179-196
- **BYRON, P.** *Adv. Drug. Del. Rev.,* 1990, vol. 5, 107-132

- **PATTON, J.S et al.** *J Controlled Release,* 1994, vol. 28, 79-85
- **DAMMS, B ; BAINS, W.** *Nature Biotechnology,* 1996
- **NIVEN, R.W et al.** *Pharm. Res,* 1995, vol. 12 (9), 1343-1349
- **KOBAYASHI, S et al.** *Pharm. Res,* 1996, vol. 13 (1), 80-83
- **LIU, R et al.** *Biotechnol. Bioeng,* 1991, vol. 37, 177-184
- **MUMENTHALER, M et al.** *Pharm. Res,* 1994, vol. 11, 12-20
- **DAMMS, B ; W. BAINS.** *Nature Biotechnology,* 1996
- **KOBAYASHI, S et al.** *Pharm. Res.,* 1996, vol. 13 (1), 80-83
- **TIMSINA, M et al.** *Int. J. Pharm,* 1994, vol. 101, 1-13
- **GONDA, I.** Topics in Pharmaceutical Sciences. Medpharm Scientific Publishers, 1991, 95-117
- **Y.-F. MAA et al.** spray-drying of air-liquid interface sensitive recombinant human growth hormone. *Journal of Pharmaceutical Sciences,* February 1998, vol. 87 (2), 152-159
- **K. MASTERS.** Spray Drying Handbook. John Wiley & Sons, 1984
- **KAWAGUCHI, H et al.** *Biomaterials,* 1986, vol. 7, 61-66
- **KRENIS, L.J ; STRAUSS, B.** *Proc. Soc. Exp. Med.,* 1961, vol. 107, 748-750
- **RUDT, S ; MULLER, R.H.** *J. Contr. Rel.,* 1992, vol. 22, 263-272
- **EDWARDS, D.A.** *J. Aerosol Sci,* 1995, vol. 26, 293-317
- Physico-chemical principles in aerosol delivery. **GONDA, I.** Topics in Pharmaceutical Sciences. Medpharm Scientific Publishers, 1991, 95-117
- **HEYDER, J et al.** *J. Aerosol Sci,* 1986, vol. 17, 811-825
- **VISSER, J.** *Powder Technology,* vol. 58, 1-10
- Aerosol dosage forms and formulations. **MOREN ; MOREN et al.** Aerosols in Medicine. Principles, Diagnosis and Therapy,. Esevier, 1985